# EUROPÄISCHE PATENTANMELDUNG

(11) **EP 2 949 635 A1**
(43) Veröffentlichungstag der Anmeldung: **02.12.2015**
(21) Anmeldenummer: 14001870.6
(22) Anmeldetag: 28.05.2014
(51) Int. Cl.: C07C 1/24

(54) **Verfahren zur Herstellung von Produktolefinen durch katalytische Dehydratisierung geeigneter Reaktanden**

(71) Anmelder: Linde Aktiengesellschaft, 80331 München (DE)
(72) Erfinder: Winkler, Florian, 81371 München (DE); Rehak, Petra, 01277 Dresden (DE)
(74) Vertreter: Fischer, Werner

(57) **Zusammenfassung**

Die Erfindung betrifft ein Verfahren zur Herstellung von Produktolefinen durch katalytische Dehydratisierung geeigneter Reaktanden, welches die Schritte eines Einspeisens eines Eduktstroms, welcher eine Alkohol-Wasser-Mischung, umfasst, in eine Dehydratisierungseinheit, wobei die Alkohol-Wasser-Mischung zumindest einen Alkohol und Wasser aufweist, und einer Umsetzung der im Eduktstrom enthaltenen Reaktanden in der Dehydratisierungseinheit durch katalytische Dehydratisierung zu einem Mischreaktionsproduktstrom umfasst, wobei die Dehydratisierungsbedingungen in der Dehydratisierungseinheit derart gewählt werden, dass die Dehydratisierung des Alkohols nur eine geringe Umsetzung des mindestens einen eingesetzten Alkohols zu dem gewünschten wenigstens einen Produktolefin aufweist, so dass der nicht reagierte wenigstens eine Alkohol im Mischreaktionsproduktstrom einen Alkoholgehalt im Bereich von 10 Gew% - 80 Gew%, insbesondere im Bereich von 15 Gew% - 60 Gew%, besonders bevorzugt im Bereich von 20 Gew% - 40 Gew%, aufweist.

## Beschreibung

Die Erfindung betrifft ein Verfahren zur Herstellung von Produktolefinen durch katalytische Dehydratisierung von geeigneten Reaktanden, insbesondere durch katalytische Dehydratisierung von Alkoholen und Alkoholgemischen.

Die Dehydratisierung von Alkoholen zu Produktolefinen durch Abspaltung von Wasser in Gegenwart eines Katalysators ist eine bekannte Reaktion. So kann beispielsweise durch Dehydratisierung von Butanol 1-Buten (in der Literatur auch als 1-Butylen bezeichnet) als Hauptprodukt und 2-Buten (bzw. 2-Butylen) als Nebenprodukt hergestellt werden. Bei der Dehydratisierung handelt es sich um eine stark endotherme Reaktion.

Die US 2011/0213104 A1 beschreibt ein Verfahren zur Herstellung von Ethylen-Butylen-Copolymeren aus nachwachsenden Rohstoffen. Dabei wird das Ethylen mittels einer Dehydratisierungsreaktion aus Ethanol, welches durch eine Fermentation von Zucker bereitgestellt wird, erzeugt. Weiterhin wird Butylen mittels einer Dehydratisierungsreaktion hergestellt, wobei der Ausgangsstoff Butanol durch eine Fermentation von Zucker oder durch eine chemische Reaktion des oben erwähnten Ethanols bereitgestellt wird. Alternativ kann Butylen durch eine Dimerisierungsreaktion des durch die oben beschriebene Dehydratisierungsreaktion bereitgestellten Ethylens hergestellt werden. Dabei sind die Reaktionsbedingungen der Dehydratisierungsreaktion des Butanols so gewählt, dass eine Selektivität von 77,5% 1-Butylen und 20% 2-Butylen (bezogen auf die eingesetzte molare Menge des Butanols) im Endprodukt erreicht wird. Weiterhin wird eine Trennung der aus dem Butanol erhaltenen Dehydratisierungsprodukte mittels einer Destillation beschrieben, wobei nicht abreagiertes Butanol abgetrennt und dem Dehydratisierungsreaktor zugeführt werden kann.

Der Erfindung liegt die Aufgabe zu Grunde, ein wirtschaftliches Verfahren zur Herstellung von Produktolefinen durch katalytische Dehydratisierung geeigneter Reaktanden bereitzustellen. Insbesondere soll mit der Erfindung ein energetisch vorteilhaftes Verfahren mit einer hohen Selektivität angegeben werden.

Diese Aufgabe wird durch ein Verfahren mit den Merkmalen des unabhängigen Anspruchs 1 gelöst.

Das erfindungsgemäße Verfahren zur Herstellung von Produktolefinen durch katalytische Dehydratisierung geeigneter Reaktanden umfasst die Schritte eines Einspeisens eines Eduktstroms, welcher im Wesentlichen eine Alkohol-Wasser-Mischung umfasst, wobei die Alkohol-Wasser-Mischung zumindest einen Alkohol und Wasser aufweist, in eine Dehydratisierungseinheit, und eine Umsetzung der im Eduktstrom enthaltenen Reaktanden in der Dehydratisierungseinheit durch katalytische Dehydratisierung zu einem Mischreaktionsproduktstrom, wobei die Dehydratisierungsbedingungen in der Dehydratisierungseinheit derart gewählt werden, so dass der nicht reagierte wenigstens eine Alkohol im Mischreaktionsproduktstrom einen Alkoholgehalt im Bereich von 10 Gew% - 80 Gew%, insbesondere im Bereich von 15 Gew% - 60 Gew%, besonders bevorzugt im Bereich von 20 Gew% - 40 Gew%, aufweist. Die Gew%-Angabe (Gewichtsprozent) bezieht sich auf den Alkoholanteil im Mischreaktionsproduktstrom in Relation zu den weiteren im Mischreaktionsproduktstrom vorhandenen Verbindungen.

Das erfindungsgemäße Verfahren ermöglicht somit durch die Wahl geeigneter Reaktionsbedingungen eine niedrige Umsetzung der eingesetzten Alkohole zu dem gewünschten wenigstens einen Produktolefin. Durch eine Dehydratisierung mit einer niedrigen Umsetzung wird eine hohe Selektivität hinsichtlich der Bildung des gewünschten Produktolefins erreicht. Die als Nebenprodukte entstehenden Isomere des gewünschten Produktolefins fallen bei niedrigen Umsetzungen in einem sehr geringen Ausmaß an.

Der Mischreaktionsproduktstrom im Sinne der Erfindung umfasst das gewünschte wenigstens eine Produktolefin (Olefine werden auch als Alkene bezeichnet), optional wenigstens ein Isomer des gewünschten Produktolefins, nicht abreagierten Alkohol, während der Dehydratisierung entstandene Dialkylether, Wasser sowie weitere während der Dehydratisierung entstandene Nebenprodukte, wie beispielsweise Kohlenmonoxid, Kohlendioxid, Wasserstoff, Methan und weitere Alkane und Olefine. Der erfindungsgemäße Eduktstrom umfasst im Wesentlichen als Reaktanden Alkohole oder Alkoholgemische, insbesondere höherwertige Alkohole und Alkoholgemische. Weiterhin kann der Eduktstrom Dialkylether, die bei der Dehydratisierung entstehen und rückgeführt wurden, als Reaktanden umfassen. Dialkylether umfassen symmetrische Dialkylether (welche aus einer Reaktion zweier identischer Alkohole gebildet werden) und unsymmetrische Dialkylether (welche aus einer Reaktion zweier verschiedener Alkohole gebildet werden). Beispiele für symmetrische Dialkylether sind Diethylether, Dipropylether, Dibutylether, Dipentylether oder Dihexylether. Beispiele für unsymmetrische Dialkylether sind Butylhexylether oder Butylpentylether.

Dabei kann der Eduktstrom synthetisch hergestellte Alkohole aufweisen, die entweder Wasser enthalten oder denen Wasser hinzugefügt wurde. Alternativ kann der Eduktstrom auch über eine Fermentation bereitgestellt werden. Durch eine Fermentation von Biomasse, wie beispielsweise Zucker, können verschiedene Alkohole als Alkohol-Wasser-Mischungen erhalten werden. Vorzugsweise wird die sich bei der Fermentation bildende Alkohol-Wasser-Mischung mittels einer Anreicherungseinheit, in welcher Wasser abgetrennt wird, zu einer Alkohol-Wasser-Mischung mit einem höheren Alkoholanteil angereichert.

In einer Ausführungsform umfasst der Eduktstrom einen Alkoholgehalt von 5 Gew% - 98 Gew%, insbesondere von 40 Gew% - 96 Gew%, besonders bevorzugt von 75 Gew% - 95 Gew%.

In einer Ausführungsform der Erfindung werden Alkohole mit einem Kohlenstoffgehalt von 3 bis 8 Kohlenstoffatomen (also Alkohole aus der Gruppe der C₃-C₈ - Alkohole), insbesondere von 4 bis 6 Kohlenstoffatomen (also Alkohole aus der Gruppe der C₄-C₆ -Alkohole), eingesetzt. Die eingesetzten Alkohole umfassen sowohl lineare als auch verzweigte Alkohole. Die eingesetzten Alkohole umfassen Alkohole mit einer oder mehr (z.B. Diole, wie Butandiol, Pentandiol oder Hexandiol) funktionellen OH-Gruppen. Insbesondere werden, ohne dass das erfindungsgemäße Verfahren darauf beschränkt ist, n-Butanol, iso-Butanol, tert-Butanol, 1,4-Butandiol, 1,3-Butandiol, 2,3-Butandiol n-Pentanol, n-Hexanol sowie deren Strukturisomere (wie z.B. 2-Pentanol, 3-Pentanol, 2-Methyl-butan-2-ol, 3-Methyl-butan-1-ol, 3-Methyl-butan-2-ol, 2,2-Dimethyl-propan-1-ol, 2-Hexanol, 3-Hexanol, 2-Methylpentan-1-ol, 3-Methylpentan-1-ol, 3-Methylpentan-2-ol usw.) einzeln oder in Mischung verwendet. Besonders bevorzugt ist der Einsatz von C₄-Alkoholen, insbesondere n-Butanol.

Die Dehydratisierungseinheit ist dazu ausgebildet, aus den eingespeisten Reaktanden des Eduktstroms Alkene bereitzustellen. Dabei kann die Dehydratisierungseinheit ein oder mehrere nacheinander geschaltete Reaktoren zur Durchführung der Dehydratisierung umfassen. Die Dehydratisierung kann isotherm als auch adiabatisch ausgeführt werden.

In einer Ausführungsform der Erfindung wird im Anschluss an die Dehydratisierung der Mischreaktionsproduktstrom mittels einer Kühleinheit zur Bildung eines mehrphasigen Mischreaktionsproduktstroms abgekühlt, so dass der Mischreaktionsproduktstrom eine wässrige Phase sowie eine organisch-flüssige Phase aufweist, und wobei anschließend eine Einleitung des Mischreaktionsproduktstroms in eine Phasentrennungseinheit erfolgt, in der eine Phasentrennung der wässrigen von der wenigstens einen organisch-flüssigen Phase vorgenommen wird. Anschließend werden die wässrige Phase und die wenigstens eine organisch-flüssige Phase aus dem mehrphasigen Mischreaktionsproduktstrom abgetrennt. Die Trennung der Phasen kann beispielsweise auf einfachste Art mittels Fliehkraft, z.B. einem Separator, oder mittels Schwerkraft, z.B. Mixer-Settler Apparaturen, erfolgen.

Unter einer "wässrigen Phase" im Sinne der Erfindung ist die Phase zu verstehen, die den Hauptanteil des in der Dehydrierungsreaktion entstandenen Wassers aufweist, wobei abhängig von der Art der verwendeten Edukte die wässrige Phase ebenfalls nicht abreagierte Alkohole und bei der Reaktion entstandene Dialkylether umfasst.

Unter einer "organisch-flüssigen Phase" im Sinne der Erfindung ist eine flüssige Phase zu verstehen, die abhängig von der Art der verwendeten Edukte und den Bedingungen der Phasentrennung nicht abreagierte Alkohole, bei der Reaktion entstandene Dialkylether, Produktolefine, deren Isomere sowie Nebenprodukte umfasst. Auch kann die organisch-flüssige Phase einen geringen Anteil des in der Dehydrierungsreaktion entstandenen Wassers enthalten.

Nach der Phasentrennung wird die organisch-flüssige Phase in wenigstens eine Separationseinheit geleitet, wobei nicht abreagierte Alkohole und durch die Dehydratisierung entstandene Dialkylether derart separiert werden, dass sie einen rückführbaren Separationsstrom ausbilden, welcher dem Eduktstrom zugeführt wird.

Weiterhin wird neben dem rückführbaren Separationsstrom auch das wenigstens eine gewünschte Produktolefin und gegebenenfalls entstandene Isomere der Produktolefine) von den weiteren Nebenprodukten abgetrennt. Die vorgenannten Produktolefine bilden dabei einen Olefinstrom aus, welcher aus der Separationseinheit abgeleitet wird. Die Nebenprodukte werden in der Separationseinheit aus der organischen Phase abgetrennt und abgeleitet. Entstandene Isomere des Produktolefins/der Produktolefine können ebenfalls in den Eduktstrom rückgeführt werden oder auch aus der Anlage abgeführt werden.

Durch die Rückführung des rückführbaren Separationsstroms, welcher ja Alkohole und Diethylether enthält, wird ermöglicht, dass nicht abreagierter Alkohol und durch die Dehydratisierung entstandene Dialkylether, welche in der organisch-flüssigen Phase nach der Phasentrennung noch enthalten sind, nicht aus dem Reaktionskreislauf entfernt werden, sondern vielmehr einer weiteren Reaktion in der Dehydratisierungseinheit zur Verfügung stehen. Somit kann die Gesamtumsetzung des Alkohols zu dem gewünschten Produktolefin erhöht werden. Bei in der organisch-flüssigen Phase enthaltenen Dialkylethern handelt es sich um Zwischenprodukte, welche bei einer Dehydratisierung entstehen und welche durch eine zusätzliche Dehydratisierung zu dem gewünschten Produktolefin umgesetzt werden können. Es handelt sich somit bei diesen Dialkylethern ebenfalls um Reaktanden, die dazu geeignet sind, mittels eines Dehydratisierungsschritts zu den gewünschten Produktolefinen umgesetzt zu werden. Durch das Einleiten der Dialkylether in den Eduktstrom wird ebenfalls die Gesamtumsetzung der gewünschten Produktolefine erhöht. Die in einer Dehydratisierungsreaktion entstandenen Zwischenprodukte (also die Dialkylether) werden recycelt.

Dabei kann der Eduktstrom beim Starten der Anlage ausschließlich eine Alkohol-Wasser-Mischung aufweisen, wobei weitere Reaktanden, wie beispielsweise Dialkylether, erst nach einem ersten Durchlauf, wie oben beschrieben, in den Eduktstrom gelangen.

Im Anschluss an die Phasentrennung der wässrigen von der organisch-flüssigen Phase wird in einer Ausführungsform die wässrige Phase dem Eduktstrom über eine Aufkonzentration, in welcher der in der wässrigen Phase enthaltene mindestens eine Alkohol und durch die Dehydratisierung entstandener Dialkylether angereichert wird, zugeführt.

In dem im Stand der Technik bekannten Verfahren wird grundsätzlich das Reaktionswasser, welches nach der Dehydratisierung der Alkohol-Wasser-Mischung im Mischproduktstrom vorliegt, abgetrennt. Somit geht ein Teil des nicht umgesetzten Alkohols, welcher in dem Reaktionswasser gelöst ist, verloren. Durch das Zurückführen des in der wässrigen Phase enthaltenen, nicht abreagierten Alkohols und des in der wässrigen Phase enthaltenen Dialkylethers) stehen diese Reaktanden für eine weitere Dehydratisierung zur Verfügung und können somit zu dem gewünschten Alken umgesetzt werden. Durch diese Rückführung in den Reaktionskreislauf wird somit die Gesamtumsetzung des eingesetzten Alkohols im Hinblick auf das entstandene und gewünschte Produktolefin erhöht. Weiterhin fällt somit bei der Dehydratisierung kein Abwasser an, welches aufwändig gereinigt oder entsorgt werden muss. Auch erfolgt die Abtrennung der wässrigen Phase von der organisch-flüssigen Phase (und somit die Abtrennung der darin gelösten Reaktanden) auf einfache Art und Weise, wie beispielsweise mittels eines Dekanters, also ohne einen großen energetischen oder apparativen Aufwand.

Die Anreicherung bzw. Aufkonzentration der wässrigen Phase kann beispielsweise durch Destillation, Pervaporation oder Extraktion in einer Anreicherungseinheit erfolgen. Bei einer derartigen Anreicherungseinheit kann es sich beispielsweise auch um eine Anreicherungseinheit handeln, in welcher Alkohol aus einer Fermentation angereichert bzw. aufkonzentriert wird. Derartige Anreicherungseinheiten sind bei Verfahren, die den Ausgangsstoff (also den umzusetzenden Alkohol) aus einem Fermentationsschritt bereitstellen, normalerweise vorhanden. Somit ist es möglich, die wässrige Phase mit der Alkohol-Wasser-Mischung aus einer Fermentation zu vereinigen und einer einzigen Anreicherungseinheit zuzuführen. Die dort angereicherte Alkohol-Wasser-Mischung kann dann im Anschluss dem Eduktstrom zugeführt werden bzw. bildet dann den Eduktstrom aus und wird in die Dehydratisierungseinheit eingespeist. Damit ist der apparative und energetische Aufwand moderat.

Insbesonders gilt dies, wenn ein Fermentationsverfahren verwendet wird, bei dem eine Fermenterbrühe vorhanden ist, da dann die wässrige Phase der Fermenterbrühe zugegeben werden kann. Die Alkoholkonzentration in der wässrigen Phase ist in der Größenordnung der Alkoholkonzentration in der Fermenterbrühe und der Mengenstrom der wässrigen Phase ist im Vergleich zum Fermentierstrom relativ gering. Der Mengenstrom der wässrigen Phase ist abhängig von der Fahrweise der Anlage und erreicht häufig nur ca. 1/12 im Vergleich zum Fermentierstrom. Auch bei kontinuierlichen Fermentations-verfahren beispielsweise sind apparative Einrichtungen vorgesehen, die zur Aufkonzentration bestimmt sind. So kann beispielsweise die wässrige Phase in den Stripper bzw. direkt in die Fermenterbrühe gegeben werden. Die Einrichtungen zur Anreicherung in den verschiedenen Fermentationsverfahren sind dem Fachmann bekannt.

Alternativ kann der umzusetzende Alkohol bzw. die umzusetzende Alkoholmischung auch synthetisch hergestellt werden, wie zum Beispiel aus Synthesegas mittels geeigneter Katalysatoren. Dabei entstehen für gewöhnlich Alkoholgemische, welche entweder direkt oder nach einer Aufarbeitung (z.B. einer Destillation) dem Eduktstrom zugeführt werden. Auch hier kann die wässrige Phase an geeigneter Stelle, wie beispielsweise in die Destillation, eingespeist werden.

Die Phasentrennung der wässrigen von der organisch-flüssigen Phase ermöglicht somit eine Rückführung nicht abreagierter Alkohole und/oder entstandener Dialkylether in die Dehydrierungsreaktion auf einfache Art und Weise. Das Zurückführen des Alkohols und der Dialkylether ermöglicht eine bessere Nutzung des als Ausgangsstoff für die Herstellung eines Produktolefins verwendeten Alkohols. Dabei können der eingesetzte Alkohol und der entstandene Dialkylether durch das oben beschriebene Zurückführen der wässrigen Phase und/oder der Zurückführung des Separationsstroms in die Dehydratisierungseinheit recycelt werden. Durch das Recyceln der als Zwischenprodukte angefallenen Dialkylether und des nicht abreagierten Alkohols können insbesondere niedrige, einfacher zu erzielende Umsetzungen in der Dehydratisierungseinheit gefahren werden, ohne dass hierdurch wirtschaftliche Nachteile entstehen.

Somit wird durch das Recyceln der Zwischenprodukte (Dialkylether) und des nicht reagierten Alkohols und dem Vorliegen einer niedrigen Umsetzung in der Dehydratisierungseinheit sowohl die Selektivität als auch die Ausbeute an dem gewünschten Produktolefin erhöht.

In Ausgestaltungen der Erfindung können zumindest Teilströme der wässrigen und/oder der organisch-flüssigen Phase in den Eduktstrom rückgeführt werden. Vorzugsweise wird die wässrige Phase nach einer Anreicherung und/oder ein Separationsstrom, der aus der organisch-flüssigen Phase gewonnen wurde und die Alkohole oder/und die Dialkylether aus dem Mischreaktionsproduktstrom zumindest teilweise enthält, in die Dehydratisierungseinheit rückgeführt. So können die Alkohole oder/und die Dialkylether aus dem Mischreaktionsproduktstrom nochmals der Dehydratisierung zugeführt werden. Welche der möglichen Rückführungen verwirklicht werden, richtet sich nach der Zusammensetzung des Mischreaktionsproduktstroms und folgt wirtschaftlichen Erwägungen.

Neben der organisch-flüssigen Phase und der wässrigen Phase kann bei der Dehydrierung zusätzlich auch eine organisch-gasförmige Phase entstehen. Ob eine organisch-gasförmige Phase entsteht hängt von der als Edukte eingesetzten Alkohole, dem Umfang der Umsetzung der Alkohole sowie den Reaktionsbedingungen der Dehydratisierung und den Bedingungen in der Phasentrennungseinheit (insbesondere Druck und Temperatur) ab. Der Anteil der in der wässrigen oder der wenigstens einen organischen Phase enthaltenen Verbindungen (wie z.B. nicht abreagierter Alkohol oder Dialkylether) ist somit abhängig von den Reaktionsbedingungen und der Art der eingesetzten Alkohole. Bezüglich einer Erläuterung der Phasentrennungsbedingungen wird auf die unteren Passagen verwiesen. Auch die Art der Abtrennung und die weitere Verwendung der abgetrennten Phasen werden dadurch bestimmt. Dies gehört zu den Standardaufgaben eines Fachmanns auf dem Gebiet der Olefinherstellung und Olefintrennung und kann von ihm ohne weiteres durchgeführt werden.

Werden beispielsweise höhere Alkohole (z.B. Butanole, Pentanole oder Hexanole) oder deren Mischungen als Edukt eingesetzt ist es möglich, dass der Mischreaktionsproduktstrom neben der wässrigen Phase eine organisch-flüssige Phase aufweist. Die organisch-flüssige Phase umfasst nicht abreagierten Alkohol, durch die Dehydratisierung entstandene Dialkylether, wenigstens ein Produktolefin und gegebenenfalls die entsprechende Isomere davon. Die wässrige Phase umfasst im Wesentlichen nicht abreagierten Alkohol.

In dieser Ausführungsform ist ebenfalls eine gasförmige Phase vorhanden, welche allerdings lediglich Nebenprodukte umfasst. Die organisch-gasförmige Phase kann abgesehen von Verunreinigungen vollständig aus Nebenprodukten bestehen, wie beispielsweise Kohlenmonoxid, Wasserstoff, Kohlendioxid, Methan und Alkane. Die organisch-gasförmige Phase kann jedoch neben den Nebenprodukten auch das Produktolefin/die Produktolefine sowie Isomere des Produktolefins/der Produktolefine enthalten. Es ist auch möglich, dass weiterhin nicht umgesetzte Alkohole und Dialkylether in der organisch-gasförmigen Phase enthalten sind.
Nebenprodukte können in der Phasentrennung und/oder in der Separationseinheit und/oder in einer Isomerenabtrennungseinheit abgetrennt und abgeleitet werden. Alternativ oder zusätzlich können Nebenprodukte wie CO₂ mittels einer CO₂-Wäsche vor dem Einleiten in die Separationseinheit abgetrennt werden.

Nach der Phasentrennung der wässrige, der organisch-flüssigen und der organisch-gasförmigen Phase wird die organisch-gasförmige Phase anschließend in eine Separationseinheit zur Separation einzelner Komponenten geleitet. Dies kann beispielsweise mittels eines Kompressors erfolgen. In der Separationseinheit wird das Produktolefin/die Produktolefine (und gegebenenfalls entstandene Isomere dessen/deren) abgetrennt, wobei ein Olefinstrom ausgebildet wird, welcher aus der Separationseinheit abgeleitet wird. Mit der wässrigen Phase und der organisch-flüssigen Phase wird verfahren, wie weiter oben beschrieben.

Wird beispielsweise eine Alkohol-Mischung aus niederen Alkoholen (z.B. n- bzw. isoPropanol) und höheren Alkoholen (z.B. Butanole, wie n-, iso- und/oder tert-Butanol und/oder z.B. Pentanole, wie 1-Pentanol und Isomere davon) als Edukt verwendet, weist der Mischreaktionsproduktstrom - neben der wässrigen Phase - eine organischflüssige Phase und eine organisch-gasförmige Phase auf. Die wässrige Phase weist dabei nicht abreagierte Alkohole (z.B. Butanole oder Propanole) und einen Anteil an Dialkylether auf. Die organisch-flüssige Phase umfasst einen Anteil nicht abreagierter Alkohole, einen Anteil an Dialkylether und wenigstens ein Produktolefin, wie z.B. 1-Buten, sowie gegebenenfalls entsprechende Isomere des wenigstens einen Produktolefins. Die organisch-gasförmige Phase umfasst wenigstens ein Produktolefin (z.B. Propen) sowie Edukte (Alkohole) und Dialkylether.

Insbesondere bei der Verwendung höherer Alkohole kann durch das Abkühlen des Mischreaktionsproduktstroms und der damit verbundenen Bildung eines zweiphasigen flüssigen Mischreaktionsproduktstroms in vorteilhafter Weise der zweiphasige Mischreaktionsproduktstrom auf einfache Art und Weise mittels der Phasenabtrenneinheit in jene wässrige Phase und jene organisch-flüssige Phase getrennt werden. Die Abtrennung der wässrigen Phase von der organisch-flüssigen Phase kann beispielsweise mittels eines Dekanters erfolgen.

Das erfindungsgemäße Verfahren ermöglicht durch die Wahl geeigneter Reaktionsbedingungen eine niedrige Umsetzung der eingesetzten Alkohole, welche zu einer erhöhten Selektivität der gewünschten Produktolefine führt. Dabei bilden sich nahezu keine oder kaum Isomere der Produktolefine. Weiterhin ermöglicht die Phasentrennung eine Rückführung nicht abreagierter Alkohole oder entstandene Dialkylether in die Dehydrierungsreaktion. Durch das Zurückführen bzw. "Recyceln" der als Zwischenprodukte angefallenen Dialkylether und des nicht abreagierten Alkohols können insbesondere niedrige, einfacher zu erzielende Umsetzungen in der Dehydratisierungseinheit gefahren werden, ohne dass hierdurch wirtschaftliche Nachteile entstehen.

Die anfallenden Isomere der Produktolefine, deren mengenmäßiger Umfang, wie bereits gesagt, meist sehr gering ist, werden entweder abgetrennt oder einer Isomerisierungseinheit zugeführt. Ferner ist es auch denkbar, die Isomere der Produktolefine in den Produktolefine zu belassen. Vorzugsweise werden die Isomere des Produktolefins in der Isomerisierungseinheit zumindest teilweise in das Produktolefin umgewandelt. Auch können die unerwüschten Isomere der Produktkolefine zumindest teilweise wieder der Dehydratisierungseinheit zugeführt werden, wo sie zumindest teilweise umgesetzt werden. Bei einer Rückführung muss jedoch darauf geachtet werden, dass sich die Isomere der Produktolefine nicht im Kreislauf anreichern.

Weiterhin kann durch die Wahl von Dehydratisierungsbedingungen, die geeignet sind, eine niedrige Umsetzung bereitzustellen, der im Stand der Technik übliche weitere Isomerisierungsschritt entfallen. Bei einer niedrigen Umsetzung fallen unerwünschte Nebenprodukte, wie beispielsweise Isomere des gewünschten Produktolefins/der Produktolefine und andere Alkane bzw. Alkene lediglich in einem sehr reduzierten Maß an. Bei einer niedrigen Umsetzung entstehen -neben dem gewünschten Produktolefin und eine nicht unbeträchtliche Anzahl an Dialkyletherverbindungen als Zwischenprodukte. Letztere können, wie beschrieben, zurückgeführt werden. Sie stehen somit einem weiteren Dehydratisierungsschritt als Reaktanden zur Verfügung. Weiterhin entsteht durch die niedrige Umsetzungsrate das gewünschte Produktolefin in einer hohen Produktreinheit. Der Olefinstrom kann in vielen Fällen ohne eine weitere Aufarbeitung einem Verbraucher zugeführt werden.

Alternativ kann der Olefinstrom auch einer Isomerenabtrennungseinheit zugeführt werden, in welcher der Olefinstrom einer weiteren Aufreinigung und Separation unterliegt. Dies kann beispielsweise mittels einer Rektifikation erfolgen. In der Isomerenabtrennungseinheit können noch vorhandene Nebenprodukte aus dem Olefinstrom abgetrennt werden. Weiterhin erfolgt in der Isomerenabtrennungseinheit eine Abtrennung des gewünschten Produktolefins von den eventuell entstandenen Isomeren des Produktolefins. Beispielsweise kann 1-Buten von eventuell entstandenem 2-Buten aus dem Olefinstrom abgetrennt werden. Das in der Isomerenabtrennungseinheit abgetrennte gewünschte Produktolefin, wie beispielsweise 1-Buten, kann einem Verbraucher zugeführt werden.

In einigen Verfahren wird das unerwünschte Isomer anschließend in einer Isomerisierungseinheit umgesetzt. In der Isomerisierungseinheit erfolgt dann eine teilweise Umsetzung des abgetrennten Isomers zu dem gewünschten Produktolefin. Beispielsweise wird 2-Buten teilweise zu 1-Buten isomerisiert. Das in der Isomerisierungseinheit entstandene Isomerengemisch kann dann - nach einer Kühlung - der Isomerenabtrennungseinheit zur Separation des gewünschten Produktolefins aus dem Isomerengemisch zugeführt werden. Dadurch kann die Ausbeute und Selektivität an dem gewünschten Produktolefin erhöht werden.

Für eine Isomerisierung wird eine zusätzliche Energie für die notwendige Isolation des gewünschten Produktolefins aus dem Isomerengemisch der Isomerisierungseinheit benötigt, da das Isomerengemisch der Isomerenabtrennungseinheit zur Isolation des gewünschten Produktolefins zugeführt werden muss. Zusätzlich benötigt die Isomerisierung Energie, da diese bei ca. 400°C betrieben wird. Da grundsätzlich die Abtrennungen in der Isomerenabtrennungseinheit in einem Temperaturbereich von 30°C bis 100°C erfolgt, müsste somit zusätzlich eine Einheit und Energie bereit gestellt werden, die das Isomerengemisch ausgehend von diesem Temperaturbereich auf ca. 400°C erhitzt.

Bei der Durchführung des erfindungsgemäßen Verfahrens ist der zu isomerisierende Anteil äußerst gering bzw. kaum vorhanden. Entfällt die Aufarbeitung der Isomere, kann der oben beschriebene apparative und energetische Aufwand vollständig entfallen. Selbst bei einer Aufarbeitung der Isomere würde bei dem erfindungsgemäßen Verfahren ein deutlich geringerer Energieaufwand notwendig sein, da der isomere Anteil auf Grund der hohen Selektivitäten und Ausbeuten deutlich geringer ist. Der notwendige Energieaufwand um die "Recycleprodukte", also beispielsweise nicht abreagiertes Butanol und der entstandene Dibutylether, auf die Dehydratisierungstemperatur zu bringen, unterschreitet den zusätzlichen Energieaufwand für eine sonst notwendige Aufarbeitung der Isomere.

Ein weiterer Vorteil der Anwendung des erfindungsgemäßen Verfahrens ist, dass der apparative Aufwand hinsichtlich der Dehydratisierungseinheit zusätzlich reduziert werden kann. In der Regel besteht die Dehydratisierungseinheit aus einer Serienschaltung von mindestens einem Festbettreaktor, wobei insbesondere grundsätzlich zwei bis drei Reaktoren notwendig sind, um einen Vollumsatz zu erreichen. Da aber bei dem erfindungsgemäßen Verfahren kein Vollumsatz notwendig ist und die Reaktionsbedingungen in der Dehydratisierungseinheit derart gewählt sind, dass eine niedrige Umsetzung erfolgt, kann vorteilhafterweise auf einen Reaktor in der Dehydratisierungseinheit verzichtet werden.

Als Katalysatoren für den Dehydratisierungsschritt werden vorzugsweise anorganische keramische Katalysatoren, insbesondere ZrO₂, Zeolithe, Al₂O₃ oder Alumosilikate, verwendet. Es können jedoch auch weitere geeignete Katalysatoren eingesetzt werden.

In einer Ausführungsform der Erfindung werden die in der Isomerenabtrennungseinheit abgetrennten Isomere in den Eduktstrom zurückgeführt. Dabei werden die so abgetrennten Isomere vorzugsweise dann dem Eduktstrom zugeführt, wenn die Dehydratisierung mittels eines Al₂O₃ oder Alumosilikat-Katalysators durchgeführt wird. Bei der Verwendung eines derartigen Katalysators erfolgt im Dehydratisierungsschritt auch eine Isomerisierung des Isomers des gewünschten Produktolefins zu dem Produktolefin. Insbesondere wird vorzugsweise in der Isomerenabtrennungseinheit abgetrenntes 2-Buten in die Dehydratisierung zurückgeleitet, um dort mittels eines Katalysators, insbesondere eines Al₂O₃-Katalysators, zu 1-Buten isomerisiert werden.

In einer weiteren Ausführungsform der Erfindung können die Isomere des gewünschten Produktolefins auch in die Dehydratisierung zurückgeführt werden, um als Wärmeträger zu fungieren oder um das Gleichgewicht zwischen dem gewünschten Produktolefin und dem entsprechenden Isomer des gewünschten Produktolefins zu beeinflussen. Dabei erfolgt insbesondere eine Beeinflussung des Gleichgewichts zwischen 1-Buten und 2-Buten zu Gunsten von 1-Buten.

Die Dehydratisierung ist derart gestaltet, dass niedrige Umsetzungen des Alkohols erfolgen. Dies wird durch die Wahl der im Folgenden diskutierten Parameter erzielt.

Vorteilhafterweise wird die Dehydratisierung bei einer Temperatur zwischen 200°C und 500°C, insbesondere zwischen 280°C und 400°C, besonders bevorzugt zwischen 300 und 360°C durchgeführt.

In einer Ausführungsform wird die Dehydratisierung bei einer Raumgeschwindigkeit (LHSV; *liquid hourly space velocity)* von 1 h⁻¹ bis 15 h⁻¹, insbesondere von 2 h⁻¹ bis 10 h⁻¹, besonders bevorzugt von 3 h⁻¹ bis 9 h⁻¹ durchgeführt.

Unter LHSV (liquid hourly space velocity) ist im Sinne der Erfindung der Quotient aus dem Feed zur Dehydratisierung (gemessen in m³/h) und dem Katalysatorvolumen (gemessen in m³) zu verstehen.

In einer Ausführungsform erfolgt die Dehydratisierung bei einem Druck in einem Bereich von 3 bar bis 30 bar, insbesondere von 5 bar bis 17 bar, besonders bevorzugt von 6 bar bis 10 bar.

In einer weiteren Ausführungsform der Erfindung erfolgt die Phasentrennung in einem Bereich von 3 bar bis 30 bar, insbesondere von 5 bar bis 17 bar, besonders bevorzugt von 6 bar bis 10 bar.

In einer Ausführungsform der Erfindung erfolgt die Separation in einem Bereich von 3 bar bis 30 bar, insbesondere von 5 bar bis 17 bar, besonders bevorzugt von 6 bar bis 10 bar.

In einer Ausführungsform liegt der Druck bei der Dehydratisierung, der Phasentrennung und/oder der Separation in einem Bereich von 3 bar bis 30 bar, insbesondere von 5 bar bis 17 bar, besonders bevorzugt von 6 bar bis 10 bar.

In einer Ausführungsform liegt der Druck bei der Dehydratisierung, der Phasentrennung und der Separation in einem Bereich von 5 bar bis 17 bar, insbesondere von 6 bar bis 10 bar, wobei als Edukte Butanole oder höhere Alkohole verwendet werden.

Die oben aufgeführten Drücke bezüglich der Dehydratisierung, der Phasentrennung und der Separation können unabhängig voneinander aus den jeweiligen Bereichen ausgewählt werden, so dass die Dehydratisierung, die Phasentrennung und die Separation bei unterschiedlichen Drücken erfolgt. Alternativ können die Drücke, ausgewählt aus den oberen Bereichen, auch die gleichen Werte für die Dehydratisierung, und/oder Phasentrennung und/oder Separation aufweisen.

In einer Ausführungsform der Erfindung erfolgt die Phasentrennung bei einer Temperatur zwischen -10°C und 90°C, insbesondere zwischen 20°C und 90°C, besonders bevorzugt zwischen 30°C und 50°C.

Vorzugsweise wird mit dem erfindungsgemäßen Verfahren im Wesentlichen ein 1-Alken, insbesondere 1-Buten, hergestellt. Weiterhin können mit dem erfindungsgemäßen Verfahren auch andere entsprechende Alkene hergestellt werden.

Im Folgenden soll die Erfindung anhand von Figuren von besonders vorteilhaften Ausgestaltungen beschrieben werden. Es zeigen:
- Fig. 1: zeigt eine Ausgestaltungsform des erfindungsgemäßen Verfahrens zur Herstellung von 1-Buten;
- Fig. 2: zeigt die Reinheit des gebildeten Produktolefins 1-Buten in Relation zu einer bestimmten Umsetzungsrate;
- Fig. 3: zeigt das erfindungsgemäße Verfahren der Figur 1 unter Berücksichtigung der energieintensivsten Schritte;
- Fig. 4: zeigt eine Ausgestaltungsform des erfindungsgemäßen Verfahrens bei Verwendung eines Alkoholgemisches umfassend niedere und höhere Alkohole (3-Phasentrennung).

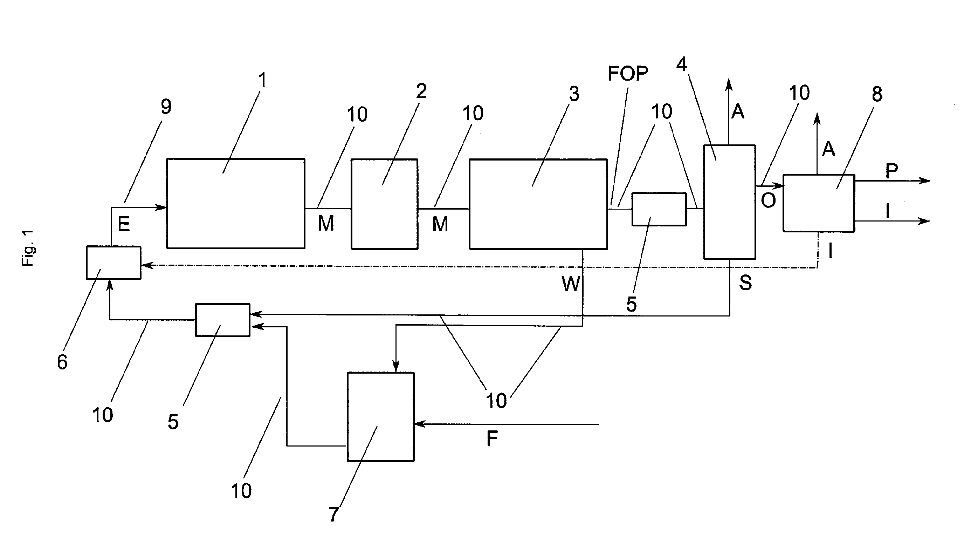

Fig. 1 zeigt eine besonders vorteilhafte Ausgestaltung des erfindungsgemäßen Verfahrens. Eine Butanol-Wasser-Mischung aus einem Fermentationsprozess (hier aus Gründen der Übersichtlichkeit nicht dargestellt) wird in eine Anreicherungseinheit 7 geleitet. Dort erfolgt eine Anreicherung des Alkoholgehalts der Butanol-Wasser-Mischung mittels einer Destillation, wobei überschüssiges Wasser aus der Abtrennungseinheit 7 abgeleitet wird. Die so angereicherte Butanol-Wasser-Mischung bildet einen Eduktstrom E aus, welcher aus der Anreicherungseinheit 7 in eine Verdichtungseinheit 5 geleitet wird.

Der Eduktstrom E wird in der Verdichtungseinheit 5, wie beispielsweise eine Pumpe, auf einen Druck von 5 bar bis 17 bar verdichtet und anschließend in eine Erhitzungseinheit 6 geleitet, wo der Eduktstrom auf eine Temperatur von 300°C bis 360°C erhitzt wird.

Im Anschluss erfolgt ein Einspeisen des verdichteten und überhitzten Eduktstroms in die Dehydratisierungseinheit 1. Die Dehydratisierung kann einen oder mehrere Reaktoren (hier aus Gründen der Übersichtlichkeit nicht dargestellt) aufweisen. In der Dehydratisierungseinheit 1 erfolgt die Dehydratisierung des Butanols. Dabei sind die Reaktionsbedingungen in der Dehydratisierungseinheit derart gewählt, dass nur eine geringe Umsetzungsrate des eingesetzten Butanols zu dem gewünschten Produktolefin 1-Buten vorliegt. Bei den vorgegebenen Reaktionsbedingungen wird bevorzugt neben dem gewünschten 1-Buten der entsprechende Dibutylether generiert. Das Isomer (2-Buten) zu dem Produktolefin 1-Buten fällt nur in einem sehr geringen Maße an. Andere Nebenprodukte, wie beispielsweise weitere C₄-Kohlenwasserstoffe können nur in Spuren beobachtet werden.

Nach der Dehydratisierung werden die Reaktionsprodukte in eine Kühleinheit 2 geleitet. Dabei ist der Druck der Dehydratisierung derart gewählt worden, dass nach der Kühlung auf ca. 40°C sich im Wesentlichen ein zweiphasiger Mischreaktionsproduktstrom M ausbildet. Es bildet sich dabei eine organisch-flüssige Phase FOP, welche im Wesentlichen nicht abreagiertes Butanol, Dibutylether, das gewünschte Produktolefin 1-Buten und sein entsprechendes Isomer 2-Buten (letzteres in geringen Mengen) umfasst. Bei der zweiten gebildeten Phase handelt es sich um eine wässrige Phase, die flüssig vorliegt und welche im Wesentlichen - aufgrund eingeschränkter Löslichkeit - in geringen Mengen Butanol und eine geringere Menge des gebildeten Dibutylethers umfasst.

Der gebildete zweiphasige Mischreaktionsproduktstrom M wird aus der Kühleinheit 2 in eine Phasentrennungseinheit 3 geleitet. In der Phasentrennungseinheit 3 erfolgt bei einer Temperatur von 40°C und einem Druck von 3 bar bis 16 bar eine Trennung der beiden flüssigen Phasen. Dies kann beispielsweise mittels eines Dekanters erfolgen. Die wässrige Phase wird in die Anreicherungseinheit 7 geleitet. Somit wird ein Teil des nicht abreagierten Butanols (und geringe Mengen des gebildeten Dibutylethers) in der Anreicherungseinheit 7 mit der Butanol-Wasser-Mischung aus der Fermentation (Fermentationsstrom F) vereinigt, dort durch Wasserabtrennung angereichert und dem Eduktstrom E anschließend zugeführt. Alternativ kann die wässrige Phase nach Durchlauf der Anreicherungseinheit 7 mit einem Eduktstrom E vereinigt werden, bei welchem der Alkohol (bzw. die Alkohole) aus Synthesegas bereitgestellt werden (hier nicht dargestellt). Es entsteht somit kein verschmutzter Abwasserstrom in dem Dehydratisierungsschritt, sondern das dort anfallende Wasser wird dem Anreicherungsschritt zugeführt. Dadurch kann das während der Dehydratisierung anfallende Wasser, welches Reaktanden enthält, dem Reaktionskreislauf wieder zugeführt werden. Lediglich aus der Anreicherungseinheit 7 abgetrenntes und im Wesentlichen reines Wasser fällt an.

Die in der Phasentrennungseinheit 3 abgetrennte organisch-flüssige Phase FOP kann über eine Pumpe in eine nachgeschaltete Separationseinheit 4 gefördert werden. In einer hier nicht dargestellten Alternative kann zusätzlich eine organisch-gasförmige Phase vorliegen. Diese kann über einen Kompressor in die Separationseinheit überführt werden. Die weiteren Schritte gelten im Wesentlichen analog.

Vorteilhafter Weise kann der Druck des Dehydratisierungsschritts so gewählt werden, dass er bereits ausreichend hoch für die Durchführung der Separationsschritte in der Separationseinheit 4 ist, womit die Verdichtungseinheit somit entfallen kann. In der Separationseinheit 4 werden die entstandenen Alkene (1-Buten und 2-Buten) von dem nicht abreagierten und in der organisch-flüssigen Phase FOP noch enthaltenen Butanol (bei höheren Alkoholen, wie z.B. Butanol, ist der Großteil des nicht abreagierten Alkohols in der Regel in der organisch-flüssigen Phase enthalten) und dem als Zwischenprodukt entstandenen Dibutylether und den weiteren Nebenprodukten abgetrennt. Das nicht abreagierte Butanol und der entstandene Dibutylether bilden einen Separationsstrom S aus, welcher in die Verdichtungseinheit 5 geleitet wird, dort mit dem aus der Anreicherungseinheit 7 stammenden angereicherten Reaktandengemisch vermischt, verdichtet und anschließend in den Eduktstrom E geleitet wird.

Weiterhin werden in der Separationseinheit 4 zusätzlich noch die in der organisch-flüssigen Phase FOP enthaltenen Nebenprodukte wie beispielsweise Kohlenmonoxid, Wasserstoff, Kohlendioxid, Methan oder Alkane abgetrennt und abgeleitet.

Die aus der organisch-flüssigen Phase FOP in der Separationseinheit 4 abgetrennten Produktolefine P (1-Buten und 2-Buten) können in eine Isomerenabtrennungseinheit 8 geleitet werden. Dort erfolgt eine weitere Abtrennung eventuell noch vorhandener Nebenprodukte (wie bereits beschrieben). Insbesondere erfolgt in der Isomerenabtrennungseinheit 8 eine Trennung des gewünschten Produktolefins 1-Buten von dem entsprechenden Isomer 2-Buten. Das isolierte 1-Buten kann einem Verbraucher zugeführt werden.

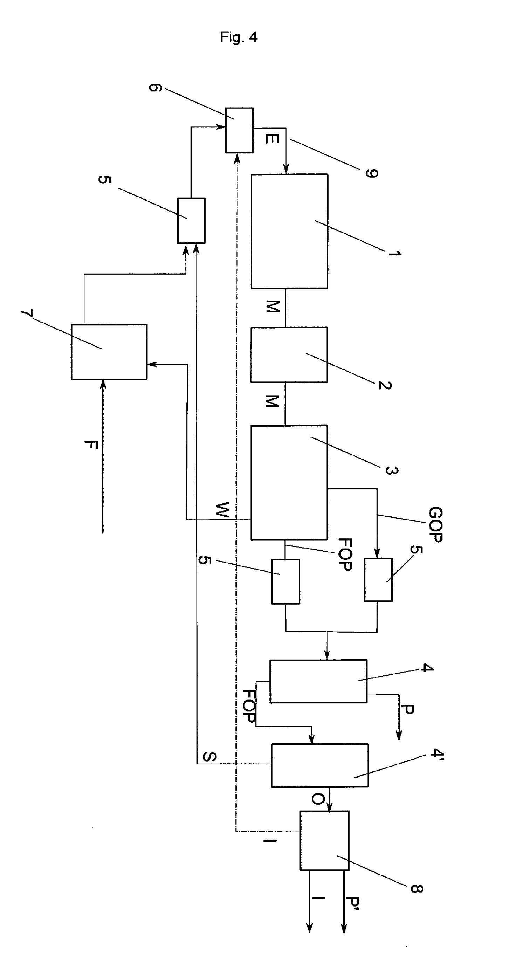

Das gemäß dem erfindungsgemäßen Verfahren entstandene 2-Buten ist in seiner Menge so gering, dass eine energieintensive Isomerisierung des 2-Butens zu dem gewünschten 1-Buten meist nicht notwendig ist. Alternativ ist es möglich (wie in Figur 4 dargestellt), dass das abgetrennte 2-Buten in eine Isomerisierungseinheit 11 geleitet wird, wobei es dort zu einem Reaktionsgemisch bestehend aus 1-Buten und 2-Buten isomerisiert wird, welches dann zu einer weiteren Abtrennung wieder in die Isomerenabtrennungseinheit 8 (nach einem Abkühlen auf die gewünschte Isomerenabtrennungstemperatur von 30°C bis 100°C) geleitet wird. Dort erfolgt wiederum eine Separation des 1-Butens und des 2-Butens, wobei das abgetrennte 2-Buten wiederum in die Isomerisierungseinheit 11 geleitet wird.

Alternativ können, wenn die Dehydratisierung mittels eines Katalysators ausgewählt aus der Gruppe von Al₂O₃ oder Alumosilikate erfolgt, die abgetrennten Isomere I dem Eduktstrom E zugeführt werden. Diese Katalysatoren sind dazu geeignet, die abgetrennten Isomere während der Dehydratisierung in die Produktolefine zu überführen (diese Alternative wird mittels einer gestrichelten Linie in der Figur dargestellt).

In einer weiteren Alternative können die Isomere, sofern die Alkohole synthetisch hergestellt wurden, einem Reformer zugeführt werden (hier nicht dargestellt).

Das erfindungsgemäße Verfahren weist keinen separat zu entsorgenden Abwasserstrom auf. Vielmehr werden der nicht abreagierte Butanolanteil und die gebildeten Zwischenprodukte (Dibutylether) über die Phasenabtrennungseinheit 3 und die Separationseinheit 4 dem Eduktstrom E zu einer weiteren Umsetzung in der Dehydratisierungseinheit 1 wieder zugeführt. Durch dieses Wiederverwenden und der geringen Umsetzung in der Dehydratisierungseinheit 1 wird eine hohe Selektivität und Ausbeute an 1-Buten erzielt. Durch die gezielte Wahl geeigneter Reaktionsbedingungen ist es mittels des erfindungsgemäßen Verfahrens somit möglich, sowohl die Selektivität als auch die Ausbeute an 1-Buten zu steigern, ohne dass dabei die Verwendung eines Isomerisierungsschrittes notwendig wäre. Mit dem erfindungsgemäßen Verfahren ist es möglich, beispielsweise die 1-Butenausbeute um ca. 20% zu steigern, wobei die Dehydratisierung bis zu einer Umsetzung von ca. 50% durchgeführt wird. Die im Stand der Technik bekannten Verfahren verfolgen im Dehydratisierungsschritt grundsätzlich eine ca. 90%ige Umsetzung. Betrachtet man die isolierten Butengemische (1-Buten und 2-Buten), so ermöglicht das erfindungsgemäße Verfahren einen 1-Butenanteil von über 95%. Im Stand der Technik wird beispielsweise (US-Patent 2011/0213104 A1) ein 1-Butenanteil von 77,5% und ein 2-Butenanteil von 20% erreicht.

Alternativ können auch andere Alkohole oder Alkoholgemische eingesetzt werden.

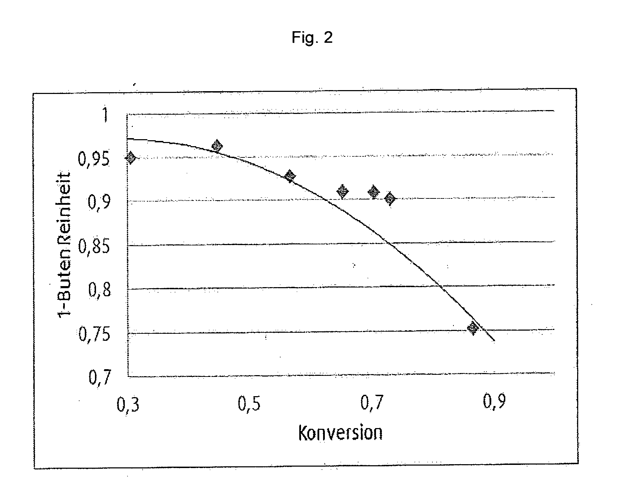

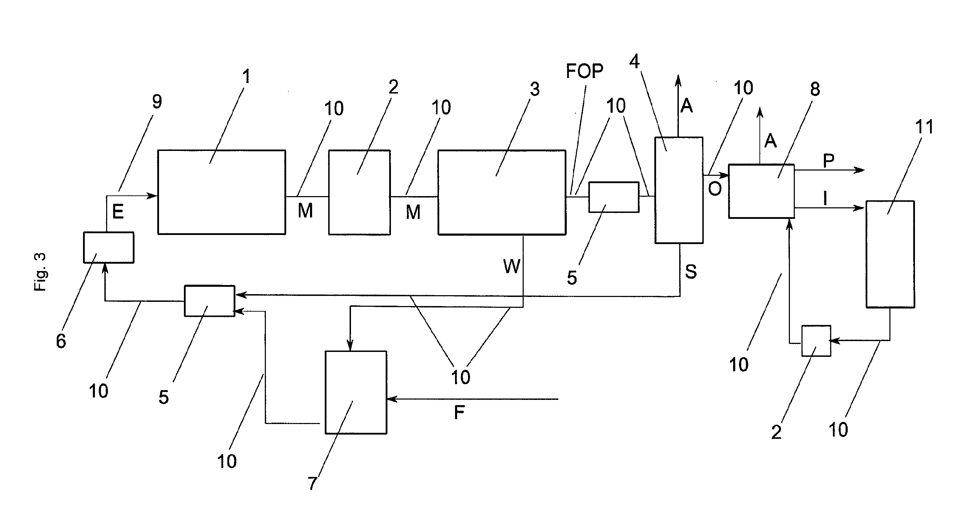

Fig. 2 zeigt die Reinheit des entstandenen Butengemischs (1-Buten und 2-Buten) nach einer Abtrennung aus einer Separationseinheit 4 in Bezug auf die in der Dehydratisierungseinheit vorliegende Umsetzung. Wie der Figur 3 zu entnehmen ist, kann bei einer Umsetzung von unter 50% eine Reinheit von über 95% an 1-Buten erzielt werden. Selbst bei einer Umsetzung von über 70% kann noch eine Reinheit von ca. 90% in Bezug auf das erhaltene gewünschte 1-Buten erzielt werden.

Die Fig. 3 zeigt das erfindungsgemäße Verfahren bei einer Verwendung einer Isomerisierungseinheit 11, wobei der notwendige energetische Aufwand berücksichtigt wurde. Bei Elementen, die mit demselben Bezugszeichen versehen sind, wird auf die Erläuterungen der Figur 1 verwiesen. Durch eine Isomerisierung fällt in der Isomerisierungseinheit 11 ein erhöhter energetischer Aufwand an, da die Isomerisierungseinheit 11 bei ca. 400°C betrieben werden muss, um eine geeignete Isomerisierung zu erzielen. Das Isomerisierungsgemisch muss dann, bevor es der Isomerenabtrennungseinheit 8 wieder zugeführt wird, auf einen Bereich von 30°C bis 100°C abgekühlt werden. Auch der zusätzliche Abtrennungsschritt des in der Isomerenabtrennungseinheit 8 bereitgestellten Isomerisierungsgemischs benötigt weitere Energie um eine Trennung, beispielsweise mittels Destillation, des gewünschten Produktolefins 1-Buten von dem entsprechenden Isomer 2-Buten bereitzustellen.

Durch Einsatz des erfindungsgemäßen Verfahrens wird der zu isomerisierende Anteil beträchtlich geringer. Dies ermöglicht eine hohe Energieeinsparung. Der zusätzliche Energieaufwand der notwendig ist, um das nach der Dehydratisierung über die Phasentrennung bzw. die Separation abgetrennte nicht abreagierte Butanol und den entstandenen Dibutylether abzukühlen, unterschreitet den Energieaufwand, der bei einer vollständigen Isomerisierung notwendig wäre.

Weiterhin kann durch das erfindungsgemäße Verfahren auch der apparative Aufwand reduziert werden. Grundsätzlich erfolgt eine Dehydratisierung in einer Serienschaltung der Festbettreaktoren, insbesondere von mindestens zwei bis drei Reaktoren, um einen Vollumsatz zu erreichen. Da erfindungsgemäß kein Vollumsatz notwendig ist, sondern eine geringe Umsetzungsrate angestrebt wird, kann in der Dehydratisierungseinheit meist auf einen Reaktor verzichtet werden.

Die Fig. 4 zeigt das erfindungsgemäße Verfahren bei Verwendung eines Alkoholgemisches aus niederen und höheren Alkoholen (3-Phasentrennung). Im Wesentlichen erfolgt das Verfahren analog zu dem in Figur 1 beschriebenen Verfahren. Vorliegend werden lediglich die Unterschiede diskutiert. Bei Elementen, die mit demselben Bezugszeichen versehen sind, wird auf die Erläuterungen der Figur 1 und Figur 3 verwiesen.

Durch die Verwendung eines derartigen Alkoholgemischs liegen nach der Dehydratisierung und Kühlung in der Phasentrennungseinheit 3 eine wässrige Phase W, eine organischflüssige Phase FOP und eine organisch-gasförmige Phase GOP vor. In der Phasentrennungseinheit 3 werden die drei Phasen voneinander getrennt.

Die wässrige Phase W wird der Anreicherungseinheit 7 zur Aufkonzentration zugeleitet und anschließend in den Eduktstrom E überführt. Die organisch-flüssige Phase FOP wird mittels einer Verdichtungseinheit 5, vorliegend eine Pumpe, und die organisch-gasförmige Phase GOP mittels einer weiteren Verdichtungseinheit 5, vorliegend einen Kompressor, in eine erste Separationseinheit 4 geleitet.

In der ersten Separationseinheit 4 werden die Produktolefine P von den Nebenprodukten getrennt und abgeleitet. Bei Bedarf können die Produktolefine P einer weiteren Auftrennung, welche die verschiedenen Produktolefine voneinander trennt, zugeführt werden.

Die verbliebene organisch-flüssige Phase FOP wird in eine zweite Separationseinheit 4' geleitet, in welcher die entstandenen und noch in der organisch-flüssigen Phase FOP enthaltenen Produktolefine P'von dem nicht abreagierten höheren Alkoholen und ggf. entstandenen Dialkylethern (und eventuell noch vorhandenen weiteren Nebenprodukten) abgetrennt werden. Die nicht abreagierten Alkohole und die entstandenen Dialkylether bilden einen Separationsstrom S aus, welcher in die Verdichtungseinheit 5 geleitet wird, dort mit dem aus der Anreicherungseinheit 7 stammenden angereicherten Reaktandengemisch vermischt, verdichtet und anschließend in den Eduktstrom E geleitet wird.

Die aus der organisch-flüssigen Phase FOP in der Separationseinheit 4' abgetrennten Produktolefine P' werden in eine Isomerenabtrennungseinheit 8 geleitet. Dort erfolgt eine weitere Abtrennung eventuell noch vorhandener Nebenprodukte (wie bereits beschrieben). Insbesondere erfolgt in der Isomerenabtrennungseinheit 8 eine Trennung des gewünschten Produktolefins P von den entsprechenden Isomeren.

Aus Gründen der Übersichtlichkeit wurde auf die Angabe des Abgasstroms A und der Ableitungssysteme 10 verzichtet. Es wird auf die Figuren 1 und 3 verwiesen.

Durch die Wahl eines geeigneten Druckes der Dehydratisierung kann mindestens ein Verdichter nach der Phasentrennung eingespart werden.

**Bezugszeichenliste**

| | |
|---|---|
| 1 | Dehydratisierungseinheit |
| 2 | Kühleinheit |
| 3 | Phasentrennungseinheit |
| 4 | Separationseinheit |
| 5 | Verdichtungseinheit |
| 6 | Erhitzungseinheit |
| 7 | Anreicherungseinheit |
| 8 | Isomerenabtrennungseinheit |
| 9 | Einleitungseinheit |
| 10 | Ableitungssystem |
| 11 | Isomerisierungseinheit |
| A | Abgasstrom |
| E | Eduktstrom |
| I | Isomer des Produktolefins |
| F | Fermentationsstrom |
| M | Mischreaktionsproduktstrom |
| O | Olefinstrom |
| FOP | organisch-flüssige Phase |
| GOP | organisch-gasförmige Phase |
| P | Produktolefin |
| S | Separationsstrom |
| W | wässrige Phase |

## Patentansprüche

1. Verfahren zur Herstellung von Produktolefinen durch katalytische Dehydratisierung, aufweisend die Schritte:
- Einspeisen eines Eduktstroms (E), welcher eine Alkohol-Wasser-Mischung umfasst, in eine Dehydratisierungseinheit (1), wobei die Alkohol-Wasser-Mischung zumindest einen Alkohol und Wasser aufweist,
- Umsetzung der im Eduktstrom (E) enthaltenen Reaktanden in der Dehydratisierungseinheit (1) durch katalytische Dehydratisierung zu einem Mischreaktionsproduktstrom (M),
**dadurch gekennzeichnet, dass**
die Dehydratisierungsbedingungen in der Dehydratisierungseinheit (1) derart gewählt werden, so dass der nicht reagierte wenigstens eine Alkohol im Mischreaktionsproduktstrom (M) einen Alkoholgehalt im Bereich von 10 Gew% - 80 Gew%, insbesondere im Bereich von 15 Gew% - 60 Gew%, besonders bevorzugt im Bereich von 20 Gew% - 40 Gew%, aufweist.

2. Verfahren nach Anspruch 1, **dadurch gekennzeichnet, dass** der Mischreaktionsproduktstroms (M) abgekühlt wird, so dass sich ein mehrphasiger Mischreaktionsproduktstrom (M) bildet, aufweisend eine wässrige Phase (W) sowie eine organisch-flüssige Phase, wobei eine Phasentrennung (3) der wässrigen Phase (W) von der organisch-flüssigen Phase durchgeführt wird.

3. Verfahren nach Anspruch 2, **dadurch gekennzeichnet, dass** zumindest Teilströme der wässrigen und/oder der organisch-flüssigen Phase in den Eduktstrom rückgeführt werden.

4. Verfahren nach Anspruch 2 oder 3, **dadurch gekennzeichnet, dass** weiterhin eine organisch-gasförmige Phase entsteht.

5. Verfahren nach einem der Ansprüche 2 bis 4, **dadurch gekennzeichnet, dass** im Anschluss an die Phasentrennung (3) der wässrigen von der organisch-flüssigen Phase
a. die wässrige Phase (W) dem Eduktstrom (E) über eine Aufkonzentration (7) zugeführt wird und/oder
b. die organisch-flüssige Phase in wenigstens eine Separationseinheit (4) geleitet wird, wobei nicht abreagierter Alkohol und durch die Dehydratisierung entstandene Dialkylether derart separiert werden, dass sie einen Separationsstrom (S) ausbilden, welcher dem Eduktstrom (E) zugeführt wird.

6. Verfahren nach einem der Ansprüche 1 bis 5, **dadurch gekennzeichnet, dass** in der Separationseinheit (4) die Produktolefine (P) und gegebenenfalls deren Isomere (I) von den Nebenprodukten und den Verbindungen, die den Separationsstrom (S) ausbilden, abgetrennt werden, wobei die Produktolefine (P) und gegebenenfalls deren Isomere (I) dann einen Olefinstrom (O) ausbilden, der aus der Separationseinheit (4) abgeleitet wird.

7. Verfahren nach Anspruch 4, **dadurch gekennzeichnet, dass** der Olefinstrom (O) in eine Isomerenabtrennungseinheit (8) zur Abtrennung der Isomere (I) der Produktolefine von den Produktolefinen (P) eingeleitet wird und die abgetrennten Isomere (I)
- einer Isomerisierungseinheit (11), oder
- der Dehydratisierungseinheit (1), oder
- einem Reformer
zugeführt werden.

8. Verfahren nach einem der Ansprüche 1 bis 7, **dadurch gekennzeichnet, dass** die Dehydratisierung bei einer Temperatur zwischen 200°C und 500°C, insbesondere zwischen 280°C und 400°C, besonders bevorzugt zwischen 300 °C und 360°C durchgeführt wird.

9. Verfahren nach einem der Ansprüche 1 bis 8, **dadurch gekennzeichnet, dass** die Dehydratisierung bei einer Raumgeschwindigkeit (LHSV) von 1 h⁻¹ bis 15 h⁻¹, insbesondere von 2 h⁻¹ bis 10 h⁻¹, besonders bevorzugt von 3 h⁻¹ bis 9 h⁻¹, durchgeführt wird.

10. Verfahren nach einem der vorhergehenden Ansprüche, **dadurch gekennzeichnet, dass** der mindestens eine Alkohol aus der Gruppe der C₃-C₈-Alkohole, insbesondere aus der Gruppe der C₄-C₆-Alkohole, ausgewählt ist, wobei insbesondere der Alkohol ein C₄-Alkohol ist.

11. Verfahren nach einem der vorhergehenden Ansprüche, **dadurch gekennzeichnet, dass** der Eduktstrom (E) einen Alkoholgehalt von 5 Gew% - 98 Gew%, insbesondere von 40 Gew% - 96 Gew%, besonders bevorzugt von 75 Gew% -95 Gew%, umfasst.

12. Verfahren nach einem der vorherigen Ansprüche, **dadurch gekennzeichnet, dass** der Druck bei der Dehydratisierung in einem Bereich von 3 bar bis 30 bar, insbesondere von 5 bar bis 17 bar, besonders bevorzugt von 6 bar bis 10 bar, und der Druck der Phasentrennung in einem Bereich von 3 bar bis 30 bar, insbesondere von 5 bar bis 17 bar, besonders bevorzugt von 6 bar bis 10 bar, liegt.
